# EUROPEAN PATENT SPECIFICATION

(11) **EP 1 094 824 B1**
(45) Date of publication and mention of the grant of the patent: **05.04.2006**
(21) Application number: 99927472.3
(22) Date of filing: 10.06.1999
(51) Int. Cl.: A61K 35/20, A23C 9/20, A23L 1/30, A23L 1/304, A23L 1/305, A61P 37/04

(54) **PROPHYLACTIC AND THERAPEUTIC NUTRITIONAL SUPPLEMENT FOR CREATION/MAINTENANCE OF HEALTH-PROTECTIVE INTESTINAL MICROFLORA AND ENHANCEMENT OF THE IMMUNE SYSTEM**
VORBEUGENDER UND THERAPEUTISCHER NAHRUNGSMITTELZUSATZ ZUR SCHAFFUNG/ERHALTUNG EINER DIE GESUNDHEIT SCHÜTZENDEN DARMMIKROFLORA UND ZUR STÄRKUNG DES IMMUNSYSTEMS
COMPLEMENT NUTRITIONNEL PROPHYLACTIQUE ET THERAPEUTIQUE DESTINE A LA CREATION/AU MAINTIEN D'UNE MICROFLORE INTESTINALE PROTECTRICE DE LA SANTE ET AU RENFORCEMENT DU SYSTEME IMMUNITAIRE

(30) Priority: 10.06.1998 US 95383
(43) Date of publication of application: 02.05.2001
(73) Proprietor: Crum, Albert B., Brooklyn Heights, NY 11201 (US)
(72) Inventor: Crum, Albert B., Brooklyn Heights, NY 11201 (US)
(74) Representative: Ebner von Eschenbach, Jennifer
(86) International application number: PCT/US1999/013218
(87) International publication number: WO 1999/064022

(56) References cited:
- DE-C- 4 413 839
- US-A- 4 377 569
- US-A- 4 512 977
- US-A- 4 784 852
- US-A- 5 456 924
- US-A- 5 531 989
- US-A- 5 645 834
- US-A- 5 785 990
- US-A- 5 795 602
- US-A- 5 846 569

## Description

The present invention provides novel prophylactic and therapeutic nutritive compositions that are useful in the creation and/or maintenance of a health-protective intestinal bacterial flora and simultaneously in the enhancement of the immune system. More specifically, the novel nutritional supplement compositions of the present invention contain whey protein concentrates or isolates, selenium, fructooligosaccharides, and bovine or caprine colostrum.

It has recently been determined that a major compartment of the mammalian immune system, is located in the digestive tract, and it is understood that the most intimate contact with the physical world is mediated through the ingestion of foods. [N. Wagner et al., Nature, 382(6589): 366-70 (1996). Consequently, by interacting with gut-associated lymphoid tissue, ingestants may affect the entire body and overall health of the mammal. Therefore, a healthy digestive tract is important to the overall health of a mammal.

The interaction between microflora and lymphoid tissue present in the gastrointestinal tract, and how their relationship effects overall health, is currently of great interest to the medical community. Certain groups of microflora are known to produce toxins that have adverse health effects. Certain other bacteria, however, are understood to be advantageous inhabitants of the intestinal tract of mammals. There-fore, it is desirable to manipulate the composition of gastrointestinal flora towards more beneficial bacterial species.

Diet is known to influence bacterial species composition in the gastrointestinal tract. The maintenance or enhancement of beneficial microflora may be accomplished in two principal ways. First, one may simply ingest beneficial exogenous bacteria, called "probiotics," in order to incorporate them into the gastrointestinal milieu. The other alternative is to ingest substances, called "prebiotics," that are known to support the growth of beneficial bacteria already present in the gastrointestinal tract.

Regardless of the method used to enhance beneficial bacterial flora in the gut, the results of doing so include improvement of lactose absorption and tolerance, control of intestinal infection a check on adverse overproliferation of cells lining the gastrointestinal tract, anti-cholesterolemic effects, and improvement in gastrointestinal motility. [S. E. Gilland, J. Dairy Sci., 72:2483-94 (1989)]. By ingesting compounds that help enhance the growth of beneficial bacteria, these effects may be achieved. Such beneficial bacteria include lactobacilli, which are understood to produce antibiotic-like substances that inhibit gram-positive infection, inhibit other disease causing bacteria such as Shigella sonnepi, and may possess anti-overproliferation and anti- cholesterolemic effects.

Another beneficial bacteria is Bifidobacteria, which is understood to have suppressive effects on disease-causing Escherichia coli and Clostridium perfringens. Bifidobacteria also produce acetic acid and lactic acid which lowers intestinal pH and inhibits the growth of other deleterious bacteria, and may be involved in preventing the overproliferation of cells in the colon. [D. Gallagher et al., J. Nutr., 126:1362-71 (1996)]. In addition, Bifidobacteria stimulate the production ofB vitamins, such as vitamin B₁₂, that are often deficient in people suffering from gastric disorders or inadequate ingestion of vitamin B₁₂ due to vegetarianism or poor diet.

Whey is the curd-free portion so milk that is left over from the production of cheese - - a by-product in the form of an amalgamation of low molecular weight proteins that remains after the casein fraction is removed with the curd. No more than about twenty years ago, whey protein was still little more than a waste by-product to the dairy industry, the only use of which was as animal feed by a handful of innovative cattle ranchers. Some dairy farmers also understood that whey was a good source of low-fat protein, not only for animals, but also for humans.

In recent years, however, science has finally caught up with traditional wisdom and discovered the many health benefits of whey protein. With the advent of modem food technology, whey protein has been concentrated and refined into whey protein concentrate (WPC), containing 30-85°to protein, and whey protein isolate (WPI), containing 90% or more of extremely low-fat whey protein.

Athletes were among the first people to appreciate these new low-fat protein sources - - nearly fat-free whey protein concentrate and isolates helped athletes gain lean muscle mass, while simultaneously providing a source of energy for strenuous sports activities. However, athletes are not the only ones to benefit from whey protein. A high percentage of AIDS and cancer patients are suffering from chronic diarrhea and malabsorption of nutrients. They are in danger of succumbing to the terrible wasting syndrome (cachexia) that is all too often the actual cause of death. In such cases, WPC or WPI can often stop the diarrhea, help them gain weight, and help prolong life. [G. Bousnous et al., Clinical & Investigative Med., 16(3) :204-209 (June 1993)]. Similarly, many of the elderly are malnourished and underweight due to a tendency to have little appetite, poor diet, and digestive problems. Hence, the elderly also benefit from food supplements like WPC or WPI that do not burden weakened digestive systems with food products that contain hard-to-digest fats and oils, or empty sugar calories, as in some nutritional drinks, foisted on them by intensive advertising.

On the other hand, WPC or WPI can also be used to lose weight, if combined with a low-calorie diet. It can even be used to replace a meal, as for instance, with professional people who ingest a whey protein drink in the office, instead of going out for lunch.

Aside from the purely nutritional properties and effects of whey protein, including weight control (gaining or losing weight), it is now understood that WPC and WPI are able to enhance the immune system due to the presence of certain "whey protein fractions," including serum albumin and the immunoglobulins which have immunomodulating effects. Immunomodulating effects refer to those actions that keep the immune system at homeostasis - - i.e., neither activating nor suppressing it beyond that which is beneficial to the body as a whole. There are four categories of protein fractions in whey protein. Those skilled in the art understand that so-called "major" protein factors of whey protein concentrates or isolates are, in order of prevalence, β-lactoglobulin (β-Lg), α-lactalbumin (α-La), bovine serum albumin (BSA), and the immunoglobulins in their entirety (including all five classes), each of which is known to be an important part of the immune system. [C. V. Morr and E. Y. Ha, Crit. Rev. in Food Sci. and Nutr., 33(6) :431-476 (1993)].

WPC and WPI also contain a number of so-called "minor" whey protein factors that may have important non-nutritional properties. [C. V. Morr and E. Y. Ha, cited above; P. F. Fox, Developments in Dairy Chemistry, Fox, P. F., ed., Elsevier Applied Science, New York (1989)]. These "minor" whey protein factors include lactoferrin, lactophorin, lacto-peroxidase, and lysozyme, which are also understood to have antibacterial and other biological functions. [B. Reiter, Int. J. Tissue React., 1:87 (1983); B. Reiter, Developments in Dairy Chemistry, 281, Fox, P. F., ed., Elsevier Applied Science, New York (1985); C. Kanno, J. Dairy Sc., 72:883 (1989)]. Whey proteins are a collection of different types of proteins - - each of which has a lower molecular weight than those in the casein fraction removed during cheese processing. Whey proteins include many proteins that have been characterized (such as those identified above) as well as numerous others whose physiological functions are not yet fully understood. For example, as mentioned above, the known proteins in whey protein include lysozyme and lacto-peroxidase, each of which are known to have important anti-bacterial functions. Lactoferrin is an iron-binding glycoprotein that has broad antimicrobial properties and may have immunomodulatory effects as well. One aspect of the antimicrobial effect of lactoferrin stems from its ability to chelate iron from pathogenic bacteria, thereby preventing their propagation. There is further evidence that, in addition to its iron-binding capacity, certain structural domains in lactoferrin process a broad range of antimicrobial properties. [M. Tomita et al., Adv. Exp. Med. Biol., 357:209-18 (1994)]. Although it is understood that lactoferrin exerts a bacteriostatic effect on beneficial and pathogenic bacteria, it has recently been shown that harmful bacteria, such as E. Coli, are more susceptible to lactoferrin's bacteriostatic effect than Bifidobacteria. [W. Bellamy et al., J. Appl. Bacteriol., 73 :472-79 (1992)].

Aside from the "major" and "minor" proteins, WPC also contains a variety of beneficial nutritional components, including ash, nonprotein N compounds (nitrogen-containing compounds), lipids, lactose, phospholipids, as well as trace amounts of sodium, potassium, calcium, magnesium, and phosphorus. [C. V. Morr, and E. A. Foegeding, Food Technol., 44:100 (1990)]. Nonprotein N compounds include products of protein catabolism that are ultimately converted to urea and excreted, such as blood urea nitrogen (BUN) - - the form of urea that is transported through the bloodstream to the kidney for excretion. Thus, whey protein contains a host of proteinaceous components and other constituents, including vitamins and minerals, that are known to be of critical nutritional importance in humans and animals. Whey protein is also a concentrated source of amino acids that are known to effect glutathione levels.

Glutathione is a sulfur-containing tripeptide (τglu-cys-gly) that is pivotal in cellular protection against oxygen radicals and a wide spectrum of xenobiotics, and that helps maintain cellular proteins in a functional state. Glutathione is also involved in lymphocyte proliferation. It is extremely difficult to get glutathione - - a first-class free radical scavenger and detoxifier- - into immune system cells and body tissue. Yet, accomplishing this is vital, because our immune system cells, such as lymphocytes, natural killer (NK) cells, and macrophages, depend on glutathione to accomplish their toxin surveillance and detoxification job effectively. Glutathione is synthesized in a series of enzymatic steps - - the rate limiting step involving τ-glutamyl cysteine synthetase and intracellular cysteine as the rate limiting substrate. Since concentrated whey protein is a good source of cysteine, cystine and other peptides and amino acids that are related to glutathione synthesis, it is believed that cysteine, cystine, and the other nutrients in whey protein are involved in mediating the immune response through modulation ofglutathione levels by fostering glutathione synthesis. [G. Bounous, et al., Clin. and Investigative Med., 12(3) :154-161 (1989)].

FOS are a fiber-like, indigestible type of sugar, present in a variety of plants (e.g., onions, asparagus, Jerusalem artichokes and wheat), from which they can be industrially extracted by enzymatic fermentation. Commercially available FOS is therefore a wholly natural product. The impact of FOS on intestinal microflora has been extensively studied. In vitro studies with isolated bacteria have demonstrated that FOS are good substrates for the Bifidobacteria spp - - one of the beneficial species of microflora. [J. Okada, et al., Second Neosugar Research Conference, Tokyo (1984)].

FOS are short- and medium-length chains of β-D fructans in which fructosyl units are bound by a β 2-1 glycosidic linkage. Depending on chain length, as defined by the number of osyl units (called the degree of polymerization), FOS are named oligofructose or insulin, the latter having a higher degree of polymerization. FOS are prebiotics since they selectively encourage the growth of advantageous gastrointestinal bacteria, such as Bifidobacteria.

According to current nutritional knowledge, colostrum represents a unique combination of beneficial nutrients, including carbohydrate, fat, and amino acids. Colostrum also contains natural vitamins and minerals, which, although not abundant in quantity, are highly bioavailable. At a minimum, the presence of even a relatively small amount of colostrum, as present in the compositions of the present invention described below, can therefore be expected to provide considerable nutritional enrichment.

Colostrum, however, has many health-promoting properties beyond and apart from its purely nutritional ones. For instance, it also supplies the neonate with several types of growth-promoting factors such as epidermal growth factor that induces synthesis of epidermal cells, transforming growth factor that induces collagen synthesis, wound healing and bone-resorption, and insulin-like growth factor (IGF-1) which induces epithelial cell proliferation and is necessary for growth and repair of healthy tissue. [C. E. Grosvenor, et al., Endocrine Reviews, 14(6) :710-728 (1992s); G. Carpenter, Science, 210:198 (1980); D. A. Cox, and R. R. Buerk, Eur. J. Biochem., 197:353-58 (1991); and P. G. Campbell, and C. R. Baumrucker, J. Endocrinol., 120:21-29 (1989)]. These effects of the various growth factors, whether originating from bovine, caprine or human breast milk, seem to be non-species specific and can hence be assumed to apply to humans. [S. M. Seyedin et al., J. Biol. Chem., 261 (13): :5693-95 (1986)]. Recent research also indicates that the growth factors in colostrum tend to survive the environment of the gut and can thus be is expected to exert their important functions in both neonates and adults. [C. E. Grosvenor et al., cited above].

Colostrum also contains proline-rich protein (PRP) that induces growth and differentiation or resting B lymphocytes, which is integral in the development of immune response. B lymphocytes are referred to as "resting" during their inactive phase, i.e., while not responding to stimulation by an antigen. In addition, PRP is understood to aid in the maturation ofthymocytes, originating from the thymus - - a crucial organ for the early development of the immune system. PRP is further understood to aid in the generation of T-suppressor and T-helper cells, demonstrating that it can have either a stimulating or suppressing effect on immune response. [K. Staroscik et al., Immunol., 20(12) :1277-82 (1983)].

Colostrum is further understood to contain a chromium-binding substance with properties similar to those of glucose tolerance factor (GTF) - - a trivalent chromium compound naturally occurring in Brewer's yeast that is understood to improve glucose tolerance and act with insulin in promoting normal glucose utilization.

Selenium is a trace element essential to normal metabolic functions. The level of daily selenium intake, either by supplementation or in certain foods, should be between about 50 µg and about 200 µg. Some of the suggested functions of selenium in the body include immune system stimulation, detoxification ofperoxidized fats and heavy metals, anti-inflammatory properties, and anti-proliferation properties. Extremely high doses of selenium, however, may have the opposite effect and cause the proliferation of cells.

Selenium is understood to play a role in glutathione peroxidase activity. The glutathione peroxidase enzyme, a potent antioxidant, is made up of four identical subunits, each containing selenocysteine in its primary structure. Hence, the presence of selenium is required for the proper functioning of glutathione peroxidase to oxidize free radicals, such as hydrogen peroxide. [M. E. Shils, et al., Modem Nutrition in Health & Disease, 1:242-250 (1994)]. Selenium is known to be involved in the metabolism of thyroid hormones that are involved in the immune response.

Because of the above-described properties, WPC/WPI is widely used as a functional and nutritional ingredient in medical, pharmaceutical, and human food products, e.g., infant formula, health foods and drinks, and frozen foods. [Anon., Am. Dairy Prod. Inst., Chicago (1992)). In addition, the use of whey protein as a source of biologically active components such as α-La, β-Lg, BSA, immunoglobulins, lactoferrin, and lactoperoxidase has been increasingly recognized. [B. Horton et al., J. Dietary Sci., 78:2584-89 (1995)]. Unfortunately, certain whey processing techniques which have been used in the past and which are still extensively used in the food industry (such as certain kinds of pasteurization, aeration, vacuum evaporation, and spray drying) can cause various levels of denaturation of delicate serum albumins, immunoglobulins, and other whey protein factors, thereby effecting their immune-enhancing capabilities. Although this results in products that remain a good source of non-meat protein, these products are not likely to strongly enhance the immune system. However, even partially denatured whey protein may provide a certain degree of immune-enhancement, as evidenced by increased longevity in an animal model. [D. F. Birt et al., J. Nutr., 112:2151-60 (1982)].

FOS are known to serve as substrate (i.e., food) or Bifidobacteria that make up part of the intestinal microflora. [M. S. Alles et al., Brit. J. Nutr., 76:211-221 (1996); F. R. J. Bornet, Am. J. Clin. Nutr., 59(suppl) :7635-9S (1994); F. Briet et al., Euron. J. Clin. Nutr., 49:501-507 (1995). FOS have also been shown to have a decided bifidogenic effect (i.e., having the beneficial effects of Bifidobacteria) in physiological systems like the human body. [H. Hidaka et al., Bifidobacteria Microflora, 5:37-50 (1986); H. W. Modler et al, cited above]. FOS have also been shown to reduce the occurrence of colon tumors and concomitantly developed gut-associated lymphoid tissue in mice, thereby counteracting the advanced stages of colon carcinogenesis. [F. Pierre et al., Cancer Res., 57:225-28 (1997)].

Proteins present in bovine or caprine colostrum are understood to induce growth and differentiation of resting B lymphocytes. [M. Julius et al., J. lmmunol., 140(5) :1366-71 (1988)]. Colostrum is further understood to contain a protein that helps protect against infections of *E. coli.* (A. Ouwehand et al., Infection and Immunity, 63(12) :4917-20 (1995)]. Bovine or caprine colostrum may also help enhance resistance to *Cryotosporidium parvum.* [B. Watzl et a]., Am. J. Trop. Med. Hyg., 48(4) :519-23 (1993)].

As stated above, selenium is known to play a role in glutathione peroxidase activity. Selenium appears to affect glutathione's antioxidant activity by contributing to the maintenance of the mucosal barrier in the gut. [M. G. O'Riordan et al., Nutrition, 12 (Suppl. 11-12) :S82-84 (1996); T. R. Hayward et al., J. Surg. Res., 56(4) :351-355 (1994)]. It has been shown that dietary selenium increases glutathione peroxidase activity, for example, by reducing susceptibility to lipid peroxidation of plasma and low-density lipoprotein in kidney transplant recipients. [O. Hussein et al., Transplantation, 63(5) :679-685 (1997)]. The benefits of proper levels of selenium in maintaining glutathione peroxidase levels in infants, whether breast-fed or formula-fed, has been documented. [U. Trafikowska et al., Acta Paediatr., 85(10) :1143-45 (1996)]. In addition, dietary supplementation with selenium has been shown to provide a protective effect in cells against peroxides. [Y. Kayanoki et al., J. Biochem., 119(4) :817-822 (1996)].

Many people, especially AIDS patients, have been desperately trying anything from taking free cysteine to N-acetyl-cysteine (NAC), or the drug OTZ to get glutathione into their depleted immune system cells. Such efforts have not been entirely successful since these compounds can be toxic in inappropriate dosages and since they do not always raise cellular glutathione levels anyway. [T. Bray and C. Taylor, Biochem. Pharma., 47(12) :2113-23 (1994)]. Therefore, it would be extremely advantageous to be able to raise intracellular and tissue levels of glutathione by nutritional means. This is but one function of the nutritive compositions of the present invention described in detail below.

There remains a need in the art for nutritive supplements that contain ingredients that are sufficiently bioavailable to help maintain and enhance beneficial gastrointestinal microflora, that function as a source of low-fat protein for use in building muscle and gaining weight, or alternatively, for losing weight when ingested in conjunction with a low calorie and low-fat diet, that function as a source of growth factors for the repair and growth of healthy tissue, that enhance the immune system, and that enhance glutathione levels.

The present invention overcomes the shortcomings of the prior art by providing nutritive supplement compositions that contain a novel combination of whey protein, FOS, selenium, and colostrum. Moreover, each of these ingredients is provided in a form that is bioavailable and may thus accomplish their important nutritive and immune-enhancing functions.

It is therefore an object of the present invention to provide nutritive supplement compositions comprising whey protein, FOS, selenium and colostrum.

It is a further object of the present invention to provide methods of using such nutritive compositions to protect, enhance, and maintain health-protective microflora in the intestinal tract of mammals.

It is still another object of the present invention to provide nutritive supplement compositions that are a source of low-fat protein that humans and mammals use to help build muscle and gain weight.

It is yet a further object of the present invention to provide nutritive supplement compositions that are a source of low-fat protein that, when ingested in conjunction with a low calorie and low-fat diet, may help humans and mammals lose weight.

It is still another object of the present invention to provide nutritive supplement compositions that are a source of growth factors for the repair and growth of healthy tissue.

It is a further object of the present invention to provide methods of administering the nutritive compositions of the present invention.

It is another object of the present invention to enhance the immune system of humans and mammals through the administration of such nutritive supplement compositions.

It is yet an additional object of the present invention to provide nutritive supplement compositions that enhance the levels of glutathione in human and mammalian cells and tissues.

It is still another object of the present invention to add optional ingredients to the nutritive compositions of the present invention. Various vitamins and minerals, as disclosed in the detailed description of the present invention, may optionally be added to the nutritive compositions of the present invention.

These and other objects of the present invention will be further described in the detailed description section that follows.

The nutritive compositions of the present invention are a novel combination of whey protein concentrate (WPC) and/or whey protein isolate (WPI), selenium, fructooligosaccharides (FOS), and bovine or caprine colostrum. These ingredients in combination have a prophylactic and therapeutic effect in maintaining and enhancing beneficial gastrointestinal microflora. A healthy intestinal environment will enhance the overall health of the mammal since many diseases, no matter in what part of the body they manifest themselves, actually start in the gastrointestinal tract. In addition, as described above, the nutritive supplement compositions of the present invention contain ingredients in a form that is bioavailable and may thus accomplish their important nutritive and immune-enhancing functions. The nutritive compositions of the present invention also function as a source of low-fat protein that humans and other mammals can use to help build muscle and gain weight, or alternatively, when ingested in conjunction with a low calorie and low-fat diet, may help humans and other mammals lose weight. The nutritive compositions of the present invention further function as a source of growth factors for the repair and growth of healthy tissue. Moreover, these compositions are understood to contain components that enhance the immune system of humans and mammals, and enhance the levels of glutathione, a ubiquitous intracellular antioxidant, in human and mammalian cells and tissues.

The nutritive compositions of the present invention benefit humans and animals by helping prevent afflictions that may arise from unhealthy gastrointestinal microflora. In other words, by promoting healthy bacteria in the gastrointestinal tract, the nutritive compositions of the present invention prevent the proliferation of unhealthy bacteria, thereby preventing their harmful effects. In addition, for humans and animals already suffering from an unhealthy balance of gastrointestinal bacteria, the nutritive compositions of the present invention may help ameliorate such conditions by promoting the growth of beneficial bacteria.

Moreover, the compositions of the present invention may help combat malnutrition in humans, whether due to illness or because basic foodstuffs are unavailable in certain underprivileged populations. Malnutrition and the resultant immunodeficiency plague many third world countries. These conditions are the leading cause of infant and child mortality, and play a significant role in disease in the general population and in premature death in the elderly. In particularly severe instances, there is a deficiency of all nutrients called marasmus. More often, the deficiency is primarily one of insufficient protein intake, leading to the conditions of kwashiorkor or protein-energy malnutrition (PEM). Such deficiencies are often coupled to vitamin and mineral deficiencies. These kinds of nutritional deficiencies are by no means limited to third world countries - - even industrialized nations have population segments that suffer from malnutrition of one form or another.

Malnutrition also affects persons suffering from AIDS, certain forms of cancers, and infectious and inflammatory conditions of the gastrointestinal tract, particularly the intestine (e.g., irritable bowel syndrome, Chrone's disease, and chronic gastroenteritis). In such cases, malnutrition is typically not the result of inadequate food intake, but rather from the inability to absorb foods and derive nutritional benefit. Hence, it is desirable to provide nutritive compositions that have the dual effect of providing sustenance as well as helping to improve the intestinal microflora and enhance the immune system.

The nutritive compositions of the present invention contain substantial quantities of proteins to supplement the human diet. In addition, the present compositions, when optional vitamins and minerals are added as described below, may contain from about 30% to about 500% of the Recommended Daily Allowance (RDA) or Daily Value (DV) of vitamins and minerals, as established by the National Academy of Science. Moreover, the compositions of the present invention, as more fully described below, help enhance the immune system.

As described in the above background section, whey may be in the form of powdered whey protein, or concentrated in whey protein concentrate (WPC), or whey protein isolate (WPI), the latter being the most concentrated form. Whey protein is understood to be useful in providing a low-fat energy source that is useful in weight is control, and further is known to enhance the immune system principally through whey protein fractions.

The major proteins in WPC/WPI (β-lactoglobulin, α-lactalbumin, immunoglobulins, and bovine serum albumin) are known to have immunomodulating effects. Moreover, bovine serum albumin, β-lactoglobulin, and immunoglobulin G (IgG) each contain glutamylcysteine groups - - building blocks from which immune system cells can synthesize the key antioxidant, glutathione. Glutathione, as described above, is understood to be responsible for the immuno-enhancing effect of undenatured whey protein previously described above.

Therefore, whey proteins, and preferably WPC and WPI, are an ideal and convenient food supplement for both gaining or losing weight, dependent only on the rest of the diet. In addition, WPC or WPI, particularly when combined with FOS and bovine or caprine colostrum as in the present invention, promotes gastrointestinal health. Finally, WPC or WPI, if in a relatively undenatured state, are able to promote the intracellular synthesis of physiologically effective levels of the antioxidant glutathione.

More specifically, the immune-enhancing effect of whey protein appears to derive from its ability to function, under certain favorable conditions (relative degree of non-denaturation) as a vehicle for the transport of cysteine/cystine, glutamine, and other amino acid precursors for protein synthesis. As one example, the non-essential amino acids cysteine/cystine contained in concentrated whey protein are utilized by immune system cells (i.e., lymphocytes) to synthesize the antioxidant tripeptide glutathione. In other words, it appears that enhancement of glutathione levels in the tissues from a diet of whey protein may be the underlying immune factor to have produced a number of observable immune-enhancing effects in laboratory studies with rodents, e.g., life-extension and increased resistance to carcinogens and to certain infections, as described above.

For inclusion in the nutritive compositions of the present invention, it is preferred to use WPC and WPI in their purified and undenatured form. However, even partially denatured whey protein provides a certain degree of immune-enhancement. One of the more recent and favorable processing techniques for obtaining substantially undenatured whey proteins is via cross-flow microfiltration technology (CFM) applied by Avonmore Ingredients, Inc. This technique utilizes a porcelain membrane to filter and separate denatured from undenatured small molecular weight proteins. This creates a filtrate that is highly (over 98%) undenatured. Predecessor techniques of membrane filtration involved a filtration process that could itself damage proteins as they pass through the membrane. Porcelain membranes, however, are less reactive to intact small molecular weight proteins during the course of filtration and thereby reduce potential injury to undenatured proteins while separating those that have been denatured during processing.

Since serum and tissue glutathione levels are known to decrease in degenerative diseases of aging and since many diseases are further understood to be related in part to depleted glutathione levels, the ingestion of WPC or WPI via the compositions of the present invention can reasonably be expected to increase glutathione levels in the elderly, in persons with cancer and/or post-chemotherapy or radiation therapy patients, in HIV sero-positive persons, in people with certain degenerative CNS conditions, such as Alzheimer's and Parkinson's disease, and in persons suffering from other degenerative conditions that are thought to be at least partially free radical-driven and characterized by depleted glutathione levels. Still another group that might benefit from the immune-enhancing effects of WPC or WPI, as included in the compositions of the invention, are athletes or others engaged in strenuous physical exercise.

In a preferred embodiment, the whey protein used in the compositions of the present invention are undenatured or substantially undenatured, similar to that of raw, unpasteurized milk. However, as stated above, partially denatured whey protein will retain some of its immunological function, and hence is still useful although not preferred. Commercially available whey protein concentrates and isolates vary greatly as to processing temperatures and techniques, resulting in greater or lesser relative degrees of denaturation. Commercially available forms of whey protein, WPC and WPI that are substantially undenatured include but are not limited to Provon 190 and WPC 80% [Avonmore Ingredients, Inc.].

The amount of whey protein, WPC or WPI that may be used in the compositions of the present invention will depend upon how concentrated a form of whey protein is used, and the route of administration. However, it is intended that one dosage of the compositions of the present invention (whether in liquid or solid form), whey protein, WPC or WPI may be present in the amount from about 0.01 g to about 100 g per dosage for humans and small animals, and from about 0.01 g to about 1 kg per dosage for large animals.

FOS are a fiber-like, indigestible type of sugar - - short- and medium-length chains of β-D fructans in which fructosyl units are bound by a β 2-1 glycosidic linkage - - that are present in a variety of plants (e.g., onions, asparagus, Jerusalem artichokes and wheat), from which they can be industrially extracted by enzymatic fermentation. Commercially available FOS is therefore a wholly natural product.

FOS is commercially available in the form of bulk powder [Nutraflora, Golden Technologies Company, Inc.]. It is intended that one dosage of the compositions of the present invention (whether in solid or liquid form) will contain from about 0.01 g to about 50 g of FOS per dosage for humans and small animals, and from about 0.01 g to about 500 g per dosage for large animals.

A further ingredient in the nutritive compositions of the present invention is colostrum - - the "premilk" of lactating mammals. Bovine and/or caprine colostrum are particularly useful forms of colostrum for purposes of the present invention. Colostrum represents a unique combination of beneficial nutrients, including carbohydrate, fat, and amino acids. Colostrum also contains natural vitamins and minerals, which, although not abundant in quantity, are highly bioavailable.

The health benefits of colostrum include the provision of several types of growth-promoting factors such as epidermal growth factor, transforming growth factors, insulin-like growth factor, proline-rich protein (PRP), and a chromium-binding substance with properties similar to those of glucose tolerance factor.

Oligosaccharides, naturally present in colostrum (as distinct from FOS), may prevent attachment of pathogenic bacteria (e.g., of pneumococci) to epithelial cells, and do so as specifically as antibodies. Thus, by preventing the attachment of pathogenic micro-organisms to the intestinal mucosa, these oligosaccharides fulfill a similar function to FOS (that fosters the growth of beneficial bacteria such as lactobacilli and Bifidobacteria) and help maintain healthy gastrointestinal microflora.

In a preferred form, the bovine or caprine colostrum is spray-dried, organic bovine or caprine colostrum. Such bovine or caprine colostrum is commercially available [Sterling Technology, Inc.]. It is intended that one dosage of the compositions of the present invention (whether in solid or liquid form) will contain from about 0.01 g to about 100 g of bovine or caprine colostrum per dosage in humans and small animals, and from about 0.01 g to about 1 kg per dosage in large animals. However, there is no known point of diminishing returns as far as the amount of colostrum that may be ingested is concerned. To the contrary, for therapeutic purposes, such as to fight infection or chronic diarrhea, the more colostrum the better.

As described above, selenium is understood to play a role in glutathione peroxidase levels as well as provide immuno-enhancing functions. Preferred forms of selenium for use in the nutritive compositions of the present invention are organic forms of selenium, particularly selenium amino acid chelate. This form of selenium is commercially available [Wright in Richmond, Inc., Lafayette, Louisiana; or Watson Foods Company, Inc., West Haven, Connecticut].

It is intended that one dosage of the compositions of the present invention (whether in solid or liquid form) will contain from about 20 µg to about 200 µg of selenium per dosage in humans and small animals, and from about 20 mg to about 150 mg per dosage in large animals.

Thus, the compositions of the present invention include from 0.01 g to about 100 g of whey protein, about 0.01 g to 50 g FOS, 20 µg to 200 µg selenium, and 0.01 g to 100 g bovine or caprine colostrum per dosage. One daily dosage of the nutritive compositions of the present invention may be from about 10 g per day to about 250 g taken one to three times per day for humans and small animals, for a total daily ingestion of 10 g to 750 g depending on the needs of the recipient. For large animals, one daily dosage may be from about 100 g to about 1000 g taken one to three times per day, for a total daily ingestion of about 100 g to about 3000 g depending on the needs of the recipient. In a preferred embodiment, a nutritive composition of the present invention comprises 10 g of whey, FOS, selenium, and bovine or caprine colostrum per dosage.

These preparations may be made by conventional methods. To prepare the compositions of the invention, the above-described whey protein, FOS, and bovine or caprine colostrum components may be combined in one preparation as the active ingredient in intimate admixture with a suitable carrier according to conventional compounding techniques.

Suitable carriers may take a wide variety of forms depending upon the form of preparation desired for administration, e.g., oral, sublingual, nasal, or parenteral.

In preparing the compositions in oral dosage form, any of the usual pharmaceutical media may be employed. For oral liquid preparations (e.g., suspensions, elixirs, and solutions), media containing water, oils, alcohols, flavoring agents, preservatives, coloring agents and the like may be used. Carriers such as starches, sugars, diluents, granulating agents, lubricants, binders, disintegrating agents, and the like may be used to prepare oral solids (e.g., powders, gelatin capsules, pills, and tablets) Lozenges and controlled release forms may also be used. If desired, tablets may be sugar coated or enteric coated by standard techniques. Examples of additional inactive components which provide for easier oral administration include but are not limited to beeswax, lecithin, gelatin, purified water, and glycerin.

In addition, oral dosage formulations of the compositions may be added to foods and consumed. Although any foodstuffs may be appropriate carriers for the presently disclosed compositions, dairy products such as yogurt, frozen yogurt, ice cream, cheeses, and milk are preferred.

For parenteral products, the carrier will usually comprise sterile water, although other ingredients may be included, e.g., to aid solubility or for preservation purposes. Injectable suspensions may also be prepared, in which case appropriate liquid carriers, suspending agents, adjuvants, and the like may be employed.

The nutritive supplement compositions of the present invention may optionally contain other ingredients of nutritional benefit or that aid in processing or storage of the compositions. Such optional ingredients include maltodextrin, dextrose, fructose, canola oil, com syrup solids, natural or artificial flavors or colors, guar gum, refined cellulose, dicalcium phosphate, sodium caseinate, medium chain triglycerides, dipotassiam phosphate, magnesium oxide, lecithin, ascorbic acid, inositol, choline bitartrate, vitamin E acetate, nicotinamide, calcium pantothenate, ferrous fumarate, zinc sulfate, pyridoxine hydrochloride, riboflavin, thiamine mononitrate, manganese sulfate, vitamin A palmitate, copper gluconate, folic acid, folate glutamate, biotin, sodium molybdate, potassium iodide, chromium picolinate, phytonadione, selenium amino acid chelate, vitamin D3, PABA, cyanocobalamin, glutamine, and beta-carotene. Additional ingredients known in the art to function as dietary supplements, including herbal compounds and foodstuffs, are intended to be within the scope of the present invention. These optional ingredients are preferred in the oral dosage forms in particular, and more particularly when the oral dosage form is a powder.

The nutritive compositions of the present invention may, in addition to selenium, involve the admixture of dried whey protein, FOS, and bovine or caprine colostrum, each in powdered form. Alternatively, whey protein concentrate or isolate, FOS, and bovine or caprine colostrum contained in separate containers may be combined by the user. However, regardless of the form of administration, it is important that a whey protein concentrate or isolate that has been carefully processed in such fashion as to avoid denaturation be used. As stated above, a large percentage of commercially processed whey protein concentrate and isolate on the market has been largely denatured because of processing conditions. Unless a denatured form of whey protein is used, the compositions of the present invention will have mainly nutritional value and will not be as valuable in immune system enhancement.

In an alternate embodiment, the whey protein, FOS, selenium, and bovine or caprine colostrum ingredients may be admixed in a liquid (e.g., water) and ingested. Suitable liquids into which the powdered form of the compositions of the invention may be diluted include water, milk, soy milk, or fruit juice. When in such form, the compositions of the present invention are preferably diluted into about 2.4 **ℓ** (8 oz.) of liquid per dosage. When in such form, it is preferred that at least 10 g of compositions of the present invention per dosage is used. For normal purposes, including the use of whey protein as a meal substitute in order to lose weight, one such drink (containing 10 g of the compositions of the present invention) per day is sufficient. On the other hand, if the user intends to use the liquid preparation to gain weight or for other therapeutic purposes, it is preferred that two drinks (each containing at least 10 g of the compositions of the present invention) per day are ingested along with a high complex carbohydrate diet.

The following examples illustrate the preferred embodiments of the present invention. These examples are illustrative only, and do not purport to limit the invention in any fashion.

### EXAMPLE 1

A preferred composition of the present invention for use in humans and small animals may comprise the following ingredients:

| Ingredient | Amount |
|---|---|
| whey protein | 0.01 g-100 g |
| FOS | 0.01 g-50 g |
| bovine or caprine colostrum | 0.01 g-100 g |
| selenium | 20 µg - 200 µg |

### EXAMPLE 2

A preferred composition of the present invention for use in large animals may comprise the following ingredients:

| Ingredient | Amount |
|---|---|
| whey protein | 0.01 g-1000 g |
| FOS | 0.01 g-500 g |
| bovine or caprine colostrum | 0.01 g-1000 g |
| selenium | 20 mg - 150 mg |

### EXAMPLE 3

A preferred composition of the present invention for use in humans may comprise the following ingredients:

| Ingredient | Amount |
|---|---|
| whey protein | 0.01 g-100 g |
| FOS | 0.01 g-50 g |
| bovine or caprine colostrum | 0.01 g-100 g |
| maltodextrin | 1 g-30 g |
| dextrose | 1 g-30 g |
| fructose | 1 g-30 g |
| canola oil | 0.1-0.5 g |
| com syrup solids | 1 g-50 g |
| natural flavors | 0.001 µg -1 mg |
| artificial flavors | 0.0001 µg -1 mg |
| natural colors | 0.0001 µg -10 mg |
| artificial colors | 0.0001 µg - 5 mg |
| guar gum | 0.001 g-10 g |
| refined cellulose | 0.01 g-10 g |
| dicalcium phosphate | 0.5 g-3 g |
| sodium caseinate | 0.01 g-5 g |
| medium chain triglycerides | 0.1 g - 0.6 g |
| dipotassium phosphate | 0.1 g - 5 g |
| magnesium oxide | 0.01 g - 0.9 g |
| lecithin | 0.015 g - 0.6 g |
| ascorbic acid | 0.02 g - 0.3 g |
| inositol | 0.01 g -1 g |
| choline bitartrate | 0.01 g - 1 g |
| vitamin E acetate | 10 IU-200 IU |
| nicotinamide | 0.0005 g - 0.05 g |
| calcium pantothenate | 0.01 g - 0.5 g |
| ferrous fumarate | 0.002 g - 0.3 g |
| zinc sulfate | 0.005 g - 0.06 g |
| pyridoxine hydrochloride | 0.001 g - 0.25 g |
| riboflavin | 0.0005 g - 0.1 g |
| thiamine mononitrate | 0.0005 g - 0.1 g |
| manganese sulfate | 0.0005 g - 0.1 g |
| vitamin A palmitate | 2,500 IU - 5,000 IU |
| copper gluconate | 0.0005 g - 0.006 g |
| folic acid | 400 µg - 2.5 mg |
| folate glutamate | 400 µg - 0.002 g |
| biotin | 100 µg - 600 µg |
| sodium molybdate | 15 µg - 500 µg |
| potassium iodide | 50 µg - 500 µg |
| chromium picolinate | 25 µ - 500 µg |
| phytonadione | 10 µg - 500 µg |
| selenium amino acid chelate | 20 µg - 200 µg |
| vitamin D3 | 200 IU - 800 IU |
| PABA | 1 µg - 1000 µg |
| cyanocobalamin | 0.1 µg - 1000 µg |
| glutamine | 0.2 g - 4 g |
| beta-carotene | 5,000 IU - 25,000 IU |

### EXAMPLE 4

A preferred composition of the present invention for use in humans and small animals may comprise the following ingredients:

| Ingredient | Amount |
|---|---|
| whey protein | 0.01 g - 100 g |
| FOS | 0.01 g - 50 g |
| bovine or caprine colostrum | 0.01 g - 100 g |
| glutamine | 0.2 g - 8 g |
| selenium amino acid chelate | 20 µg -200 µg |

### EXAMPLE 5

A preferred composition of the present invention for use in large animals may comprise the following ingredients:

| Ingredient | Amount |
|---|---|
| whey protein | 0.01 g -1000 g |
| FOS | 0.01 g - 500 g |
| bovine or caprine colostrum | 0.01 g -1000 g |
| glutamine | 0.2 g - 25 g |
| selenium amino acid chelate | 20 µg - 150 µg |

Many modifications may be made without departing from the basic spirit of the present invention. Accordingly, it will be appreciated by those skilled in the art that the invention may be practiced other than has been specifically described herein.

## Claims

1. A nutritive composition for the creation and maintenance of a healthy intestinal flora and for the enhancement of the immune system comprising:
(a) whey;
(b) selenium;
(c) colostrum; and
(d) fructooligosaccharides (FOS).

2. A nutritive composition according to claim 1 comprising:
(a) 0.01 g to 100 g per dosage whey;
(b) 20 µg to 200 µg per dosage selenium;
(c) 0.01 g to 100 g per dosage colostrum; and
(d) 0.01 g to 50 g per dosage fructooligosaccharides.

3. The nutritive composition according to claim 1 or claim 2 wherein the colostrum comprises bovine colostrum.

4. The nutritive composition according to any preceding claim wherein the colostrum comprises caprine colostrum.

5. The nutritive composition according to any preceding claim wherein the colostrum is spray-dried.

6. The nutritive composition according to any preceding claim wherein the colostrum is organic colostrum.

7. A nutritive composition according to any preceding claim wherein the composition additionally comprises riboflavin.

8. A nutritive composition according to claim 7 comprising:
(a) 0.01 g to 100 g per dosage whey;
(b) 20 µg to 200 µg per dosage selenium;
(c) 0.01 g to 100 g per dosage colostrum;
(d) 0.01 g to 50 g per dosage fructooligosaccharides; and
(e) 0.0005 g to 0.1 g per dosage riboflavin.

9. A nutritive composition according to any preceding claim additionally comprising glutamine.

10. A nutritive composition according to claim 9 wherein the glutamine is present in an amount of from 0.2 g to 8 g per dosage.

11. A nutritive composition according to any preceding claim wherein the selenium is a selenium amino acid chelate.

## Patentansprüche

1. Nahrungsmittelzusammensetzung für die Schaffung und Erhaltung einer gesunden Darmflora und zur Stärkung des Immunsystems, aufweisend:
(a) Molke,
(b) Selen,
(c) Colostrum und
(d) Fructooligosaccharide (FOS).

2. Nahrungsmittelzusammensetzung nach Anspruch 1, aufweisend:
(a) 0,01 g bis 100 g pro Dosierung Molke,
(b) 20 µg bis 200 µg pro Dosierung Selen
(c) 0,01 g bis 100 g pro Dosierung Colostrum und
(d) 0,01 g bis 50 g pro Dosierung Fructooligosaccharide (FOS).

3. Nahrungsmittelzusammensetzung nach Anspruch 1 oder 2, worin das Colostrum Rinder-Colostrum aufweist.

4. Nahrungsmittelzusammensetzung nach einem der vorgenannten Ansprüche, worin das Colostrum Ziegencolostrum aufweist.

5. Nahrungsmittelzusammensetzung nach einem der vorgenannten Ansprüche, worin das Colostrum sprühgetrocknet ist.

6. Nahrungsmittelzusammensetzung nach einem der vorgenannten Ansprüche, worin das Colostrum organisches Colostrum ist.

7. Nahrungsmittelzusammensetzung nach einem der vorgenannten Ansprüche, worin die Zusammensetzung zusätzlich Riboflavin aufweist.

8. Nahrungsmittelzusammensetzung nach Anspruch 7, aufweisend:
(a) 0,01 g bis 100 g pro Dosierung Molke,
(b) 20 µg bis 200 µg pro Dosierung Selen
(c) 0,01 g bis 100 g pro Dosierung Colostrum und
(d) 0,01 g bis 50 g pro Dosierung Fructooligosaccharide (FOS).
(e) 0,0005 g bis 0,1 g pro Dosierung Riboflavin.

9. Nahrungsmittelzusammensetzung nach einem der vorgenannten Ansprüche, zusätzlich aufweisend Glutamin.

10. Nahrungsmittelzusammensetzung nach Anspruch 9, worin das Glutamin in einer Menge von 0,2 bis 8 g pro Dosierung vorliegt.

11. Nahrungsmittelzusammensetzung nach einem der vorgenannten Ansprüche, worin das Selen ein Selen-Aminosäurechelat ist.

## Revendications

1. Composition nutritive pour la création et le maintien d'une flore intestinale saine pour la stimulation du système immunitaire comprenant :
(a) du petit-lait ;
(b) du sélénium ;
(c) du colostrum ; et
(d) des fructooligosaccharides (FOS).

2. Composition nutritive selon la revendication 1 comprenant :
(a) 0,01 g à 100 g par dosage de petit-lait ;
(b) 20 µg à 200 µg par dosage de sélénium ;
(c) 0,01 g à 100 g par dosage de colostrum ; et
(d) 0,01 g à 50 g par dosage de fructooligosaccharides.

3. Composition nutritive selon la revendication 1 ou la revendication 2 où le colostrum comprend du colostrum bovin.

4. Composition nutritive selon l'une quelconque des revendications précédentes où le colostrum comprend du colostrum caprin.

5. Composition nutritive selon l'une quelconque des revendications précédentes où le colostrum est séché par dispersion.

6. Composition nutritive selon l'une quelconque des revendications précédentes où le colostrum est du colostrum organique.

7. Composition nutritive selon l'une quelconque des revendications précédentes où la composition comprend en outre de la riboflavine.

8. Composition nutritive selon la revendication 7 comprenant :
(a) 0,01 g à 100 g par dosage de petit-lait ;
(b) 20 µg à 200 µg par dosage de sélénium ;
(c) 0,01 g à 100 g par dosage de colostrum ;
(d) 0,01 g à 50 g par dosage de fructooligosaccharides ; et
(e) 0,0005 à 0,1 g par dosage de riboflavine.

9. Composition nutritive selon l'une quelconque des revendications précédentes comprenant en outre de la glutamine.

10. Composition nutritive selon la revendication 9 où la glutamine est présente en une quantité de 0,2 g à 8 g par dosage.

11. Composition nutritive selon l'une quelconque des revendications précédentes où le sélénium est un chélate de sélénium et d'aminoacide.
